(19) Europäisches Patentamt
European Patent Office
Office européen des brevets

(11) Veröffentlichungsnummer: **0 241 839**
**A2**

# (12) EUROPÄISCHE PATENTANMELDUNG

(21) Anmeldenummer: **87105085.2**

(51) Int. Cl.[4]: **C07C 141/08**

(22) Anmeldetag: **06.04.87**

(30) Priorität: **14.04.86 DE 3612481**

(43) Veröffentlichungstag der Anmeldung:
**21.10.87 Patentblatt 87/43**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Schenker, Gilbert, Dr.**
**Herrmann-Hesse-Strasse 5**
**D-4006 Erkrath 2(DE)**
Erfinder: **Piorr, Robert, Dr.**
**Kieselei 12**
**D-4030 Ratingen-Hösel(DE)**
Erfinder: **Lüttge, Sabine**
**Marktfelderstrasse 30**
**D-4050 Mönchengladbach 1(DE)**

## (54) Verfahren zur Herstellung von grenzflächenaktiven Fettsäureester-Derivaten.

(57) Grenzflächenaktive Fettsäureester-Derivate werden hergestellt aus Epoxyfettsäureestern durch Umsetzung mit 1 bis 3 Mol Schwefeltrioxid pro Mol Epoxidsauerstoff und Umsetzung der dabei gebildeten Glycolsulfatgruppe mit nukleophilen Reagentien. Als nukleophiles Reagens wird bevorzugt Alkalihydroxid verwendet. Die Derivate von epoxidierten Fettsäureestern einwertiger Alkohole mit 1 bis 4 C-Atomen eignen sich als -schaumarme Tenside für Wasch-und Reinigungsmittel.

EP 0 241 839 A2

## "Verfahren zur Herstellung von grenzflächenaktiven Fettsäureester-Derivaten"

Gegenstand der Erfindung ist ein neues Verfahren zur Herstellung von Fettsäureester-Derivaten aus Epoxyfettsäureestern. Die nach dem erfindungsgemäßen Verfahren zugänglichen, neuen Stoffe haben grenzflächenaktive Eigenschaften und können als Emulgatoren und Waschrohstoffe verwendet werden.

Eine Möglichkeit, funktionelle Gruppen in das Molekül ungesättigter Fettsäureester einzuführen, besteht darin, die Doppelbindung in bekannter Weise durch Epoxidation in einen Oxiranring zu überführen und den Oxiranring mit nucleophilen Reagenzien unter Bildung von 2-Hydroxyalkylderivaten zu öffnen.

Es ist auch bekannt, Epoxide durch Umsetzung mit Schwefeltrioxid ($SO_3$) in 1,2-Glycolsulfate zu überführen. Diese Umsetzung verläuft aber bei z. B. Ethylenoxid nach J. Org. Chem. 25 (1960), Seite 864 so heftig, daß die direkte Umsetzung mit $SO_3$ zur Verkohlung führt und nur die Umsetzung mit einer $SO_3$-Dioxan-Additionsverbindung zum gewünschten Glycolsulfat führt. Nach DE-A 20 40 503 wird daher vorgeschlagen, das Epoxid zuerst mit Schwefeldioxid ($SO_2$) zum Glycolsulfit und dann mit $SO_3$ zum Glycolsulfat umzusetzen.

Es wurde nun gefunden, daß die direkte Umsetzung von Epoxyfettsäureestern mit $SO_3$ überraschend glatt unter technisch leicht zu steuernden Bedingungen ohne Verkohlung abläuft, wobei die Epoxygruppe in eine 1,2-Glycolsulfatgruppe überführt wird. Auf diese Weise wird, durch weitere Umsetzung der 1,2-Glycolsulfatgruppe mit nucleophilen Reagenzien, eine Vielzahl von Fettsäure ester-Derivaten zugänglich, die aufgrund der dabei entstehenden, wasserlöslichmachenden betasubstituierten Ethylsulfatgruppe grenzflächenaktive Eigenschaften aufweisen.

Gegenstand der Erfindung ist daher ein Verfahren zur Herstellung von Fettsäureester-Folgeprodukten aus Epoxyfettsäureestern, dadurch gekennzeichnet, daß man epoxidierte Fettsäureester mit 1 bis 2 Mol Schwefeltrioxid ($SO_3$) pro Mol Epoxidsauerstoff oder mit einer entsprechenden Menge eines $SO_3$-Luft oder $SO_3$-Inertgasgemisches bei einer Temperatur von 20 bis 100 °C zur Umsetzung bringt und die Umsetzungsprodukte weiter mit nucleophilen Reagenzien umsetzt. Bevorzugt wird die Reaktion in einem Temperaturbereich von 30 bis 80 °C durchgeführt.

Epoxyfettsäureester sind epoxidierte ungesättigte Fettsäureester. Solche Produkte sind als sogenannte Epoxidweichmacher bekannte und im Handel erhältliche Produkte. Sie werden durch Epoxidation ungesättigter Fettsäureester, z. B. nach dem in J. Am. Chem. Soc. 67, März 1945, Seiten 412 bis 414 beschriebenen Verfahren durch Umsetzung von ungesättigten Fettsäureestern mit Peressigsäure erhalten. Geeignete Fettsäureester sind solche aus ungesättigten Fettsäuren mit 14 bis 22 C-Atomen und ein-oder mehrwertigen Alkoholen mit 1 bis 6 C-Atomen, z. B. Methylester, Ethylester, Isopropylester, Ethylenglycolester, Triglyceride, Trimethylolpropanester, Pentaerytritester und Sorbitanester. Bevorzugt werden als Epoxyfettsäureester epoxidierte $C_1$-$C_4$-Alkylester der einfach ungesättigten Fettsäuren mit 16 bis 22 C-Atomen oder solcher Fettsäuregemische, die überwiegend aus einfach ungesättigten Fettsäuren mit 16 bis 22 C-Atomen bestehen, eingesetzt. Geeignet sind aber auch die epoxidierten Ester von technischen Fettsäurefraktionen, die bis zu etwa 20 Gew.-% gesättigte Fettsäuren mit 14 bis 22 C-Atomen und/oder bis zu etwa 50 Gew.-% mehrfach ungesättigte Fettsäuren, z. B. Linolsäure, enthalten.

Bevorzugt werden Epoxyfettsäureester mit einem Gehalt von 3 bis 5 Gew.-% Epoxidsauerstoff, insbesondere epoxidierter Ölsäuremethylester oder epoxidierte Methylester von Fettsäurefraktionen, die mehr als 50 Gew.-% Ölsäure enthalten.

Bei der Umsetzung der Epoxyfettsäureester mit $SO_3$ tritt als Nebenreaktion eine gleichzeitige Sulfatierung in Alphastellung zur Carboxylgruppe ein, die bei entsprechend hohem, stöchiometrischem $SO_3$-Überschuß zur Hauptreaktion wird.

Die Umsetzung verläuft dann nach folgendem Formelschema:

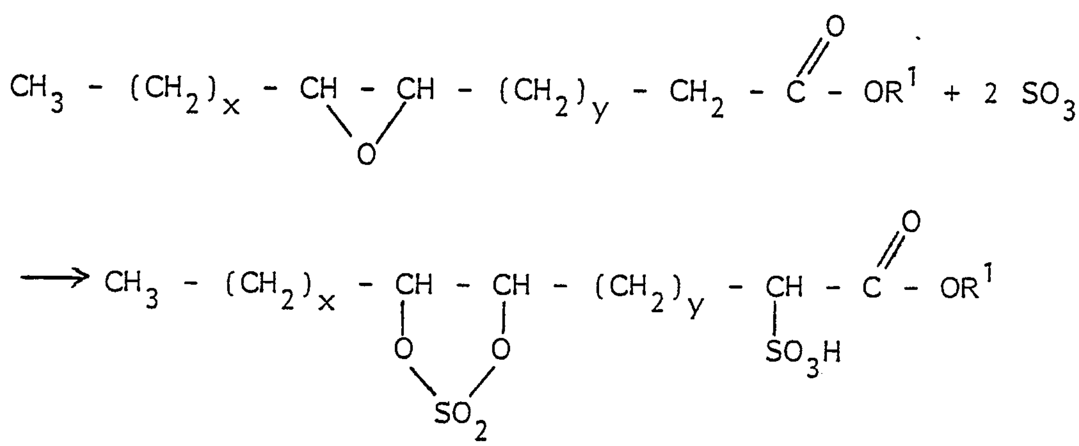

wobei $R^1$ im Falle der niederen $C_1$-$C_4$-Alkylester eine Alkylgruppe mit 1 bis 4 C-Atomen darstellt. Die Summe (x + y) ist eine Zahl von 9 bis 17. Im Falle von epoxidiertem Ölsäuremethylester ist $R^1$ = $-CH_3$ und (x + y) = 13. Aufgrund der unter den gleichen Reaktionsbedingungen eintretenden Alphasulfonierung der Fettsäureester werden Anteile von nicht epoxidierten, gesättigten Fettsäureestern unter den Reaktionsbedingungen in Alphasulfofettsäureester überführt. Anteile von nicht epoxidierten, ungesättigten Fettsäureestern werden zusätzlich an der Doppelbindung sulfoniert. Das gleiche gilt für nur teilweise epoxidierte, mehrfach ungesättigte Fettsäureester. Die Sulfonierung an der Doppelbindung verläuft im Sinne der bekannten Alphasulfonierung von Olefinen unter Ausbildung von $-SO_3H$-Gruppen in Alphastellung zur Doppelbindung, von 2-und 3-Hydroxyalkansulfonaten und Sultonen.

Die Umsetzungsprodukte der ersten Stufe des erfindungsgemäßen Verfahrens zeichnen sich dadurch aus, daß sie anstelle der Epoxygruppen nun die Glycolsulfatgruppe aufweisen. Diese ist reaktiv und läßt sich in der zweiten Stufe mit zahlreichen nucleophilen Reagenzien unter Ausbildung neuer Fettsäureester-Derivate umsetzen. Als nucleophile Reagenzien eignen sich z. B. Hydroxyl-, Alkoholat-, Phenolat-oder Carboxylationen, Ammoniak, primäre, sekundäre und tertiäre Amine. Dabei tritt eine Öffnung des Glycolsulfat-Ringes unter Ausbildung von betasubstituierten Ethylsulfatgruppierungen nach folgendem Formelschema ein

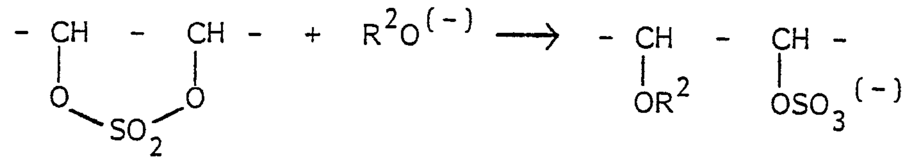

oder

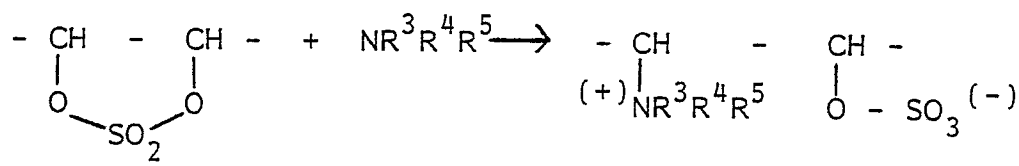

wobei $R^2$ bis $R^5$ z. B. Wasserstoff, Alkylgruppen mit 1 bis 4 C-Atomen oder $R^2$ auch eine Phenylgruppe, Benzylgruppe oder Acylgruppe mit 1 bis 4 C-Atomen sein können.

Auf diese Weise sind neue Fettsäureesterderivate zugänglich, die aufgrund der wasserlöslichmachenden Sulfatestergruppen ($-OSO_3^{(-)}$) und gegebenenfalls Sulfonatgruppen ($-SO_3^{(-)}$) grenzflächenaktive Eigenschaften aufweisen.

Bevorzugt ist die weitere Umsetzung der $SO_3$-Umsetzungsprodukte mit Hydroxylionen als nucleophilem Reagens unter hydrolytischer Aufspaltung der Glycolsulfatgruppe zu Betahydroxyethylsulfat-Gruppen. Dies läßt sich leicht dadurch erreichen, daß man das Umsetzungsprodukt mit einer wäßrigen Lösung von 1 bis 1,3 Mol Alkalihydroxid pro Mol angelagertes $SO_3$ in Kontakt bringt und bis zur Aufspaltung der Glycolsulfatgruppen zu

$$- CHOH - \underset{|}{C}HOSO_3^{(-)}$$

Gruppen erwärmt. Als Alkalihydroxid wird bevorzugt Natrium-oder Kaliumhydroxid eingesetzt. Die hydrolytische Aufspaltung der Glycolsulfatgruppen ist bei Verwendung einer ca. 50 Gew.-%igen Alkalihydroxidlösung bei 90 °C in maximal 15 bis 20 Stunden abgeschlossen. Bei höherer Temperatur und Anwendung von Druck kann die Hydrolyse beschleunigt werden.

Der Gehalt an Aniontensid läßt sich durch Bestimmung des Gehaltes an Sulfatgruppen ($-O-SO_3^{(-)}$) und Sulfonatgruppen ($-SO_3^{(-)}$) im Endprodukt nach der sogenannten Zweiphasentitrationsmethode erfassen. Diese Methode ist als DGF-Einheitsmethode H-III-10 standardisiert. Die Aufspaltung der Glycolsulfatgruppen ist abgeschlossen, wenn der Gehalt an Aniontensid nicht weiter zunimmt.

Eine Unterscheidung zwischen $-OSO_3^{(-)}$ und $-SO_3$-Gruppen läßt sich durch Zweiphasentitration nach saurer Hydrolyse treffen: Durch saure Hydrolyse lassen sich die Sulfatgruppen abspalten, so daß nach der sauren Hydrolyse nur noch der Gehalt an Sulfonatgruppen nachweisbar ist.

Die durch Aufspaltung der Glycolsulfatgruppen mit Alkalihydroxid erhältlichen grenzflächenaktiven Fettsäureester-Folgeprodukte sind für verschiedene anwendungstechnische Zwecke geeignet. Die De rivate von Epoxyfettsäureestern einwertiger Alkohole mit 1 bis 4 C-Atomen eignen sich als schaumarme Tenside für die Verwendung in Wasch-und Reinigungsmitteln.

Die nachfolgenden Beispiele sollen den Erfindungsgegenstand näher erläutern:

## Beispiele

### 1. Umsetzung bei 80 °C

In einem Labor-Standreaktor, der mit 298,5 g (ca. 1,0 Mol) eines epoxidierten technischen Ölsäuremethylester (Epoxid-Sauerstoffgehalt 3,5 Gew.-%) gefüllt und auf 80 °C erwärmt war, wurden 96,0 g (1,2 Mol) gasförmiges (aus Oleum ausgetriebenes) Schwefeltrioxid über ein Tauchrohr innerhalb von 50 Minuten eingeleitet. Das Reaktionsgemisch wurde noch weitere 30 Minuten auf 80 °C gehalten.

157,6 g des Umsetzungsproduktes (0,4 Mol) wurden dann mit 49 g (0,61 Mol) einer 50 Gew.-%igen wäßrigen Lösung von Natriumhydroxid versetzt und 20 Stunden auf 90 °C erhitzt. Zuletzt wurde mit verdünnter Schwefelsäure der pH-Wert auf 7 eingestellt und mit Wasser auf 630 g verdünnt. Das pastenförmige Tensid zeigte folgende Kenndaten:

Aktivsubstanz (Trockenrückstand): 29 Gew.-%
Aniontensid (DGF-Methode H-III-10): 0,32 mVal/g
Unsulfatiertes (DGF-Methode G-III-6b): 8,5 Gew.-%

### 2. Umsetzung bei 30 °C

In einem Labor-Standreaktor, der mit 296,3 g (ca. 1 Mol) eines epoxidierten technischen Ölsäuremethylesters (Epoxid-Sauerstoffgehalt 3,5 Gew.-%) gefüllt und auf 30 °C erwärmt war, wurden innerhalb von 50 Minuten 144,0 g (1,8 Mol) gasförmiges (aus Oleum ausgetriebenes) Schwefeltrioxid über ein Tauchrohr eingeleitet. Das Reaktionsgemisch wurde noch weitere 30 Minuten bei 30 °C gehalten.

154,1 g (0,35 Mol) des rohen Umsetzungsproduktes wurden dann mit 58,3 g (0,73 Mol) einer 50%igen wäßrigen Lösung von Natriumhydroxid versetzt und 19 Stunden auf 92 °C erhitzt. Schließlich wurde mit verdünnter Schwefelsäure der pH-Wert auf 7 eingestellt und mit Wasser auf 630 g verdünnt. Das pastenförmige Produkt zeigte folgende Kenndaten:

Aktivsubstanz (Trockenrückstand): 29 Gew.-%
Aniontensid (DGF-Methode H-III-10): 0,49 mVal/g
Unsulfiertes (DGF-Methode G-III-6b): 3,0 Gew.-%

3. Umsetzung bei 30 °C und Reaktion mit Diethanolamin

In einem Labor-Standreaktor, der mit 148 g (0,5 Mol) eines epoxidierten technischen Ölsäuremethylesters (Epoxid-Sauerstoffgehalt 3,5 Gew.-%) gefüllt und auf 30 °C erwärmt war, wurden innerhalb von 50 Minuten 40 g (0,5 Mol) gasförmiges (aus Oleum ausgetriebenes) Schwefeltrioxid über ein Tauchrohr eingeleitet. 155 g (0,4 Mol) des rohen Sulfierproduktes wurden anschließend mit 43 g (0,41 Mol) Diethanolamin neutralisiert (pH 7).

Das erhaltene Umsetzungsprodukt wurde mit 462 g Wasser versetzt (Konz. = 30 %ig) und 4 Stunden auf 90 °C erhitzt. Dabei wurde weitere 8,7 g (0,08 Mol) Diethanolamin zugesetzt, um den pH-Wert auf 7 zu halten. Das pastenförmige Produkt zeigte folgende Kenndaten:

Aniontensid (DGF-Methode H-III-10) : 0,27 m Val/g
Unsulfiertes (DGF-Methode g-III-6b) : 9,3 Gew.-%

## Ansprüche

1. Verfahren zur Herstellung von grenzflächenaktiven Fettsäureester-Derivaten aus Epoxyfettsäureestern, dadurch gekennzeichnet, daß man epoxidierte Fettsäureester mit 1 bis 3 Mol Schwefeltrioxid ($SO_3$) pro Mol Epoxidsauerstoff oder mit einer entsprechenden Menge eines $SO_3$-Luft-oder $SO_3$-Inertgasgemisches bei einer Temperatur von 20 bis 100 °C zur Umsetzung bringt und das Umsetzungsprodukt (A) weiter mit nucleophilen Reagenzien umsetzt.

2. Verfahren nach Anspruch 1, dadurch gekennzeichnet, daß als Epoxyfettsäureester epoxidierte $C_1$-$C_4$-Alkylester einfach ungesättigter Fettsäuren mit 16 bis 22 C-Atomen oder solcher Fettsäuregemische eingesetzt werden, die überwiegend aus solchen Fettsäuren bestehen.

3. Verfahren nach den Ansprüchen 1 oder 2, dadurch gekennzeichnet, daß als Epoxyfettsäureester epoxidierter Ölsäuremethylester eingesetzt wird.

4. Verfahren nach den Ansprüchen 1 bis 3, dadurch gekennzeichnet, daß als nucleophile Reagenzien Hydroxyl-, Alkoholat-, Phenolat-oder Carboxylationen, Ammoniak, primäre, sekundäre oder tertiäre Amine eingesetzt werden.

5. Verfahren nach den Ansprüchen 1 bis 4, dadurch gekennzeichnet, daß man das Umsetzungsprodukt (A) mit einer wäßrigen Lösung von 1 bis 1,3 Mol Alkalihydroxid pro Mol angelagertes $SO_3$ in Kontakt bringt und bis zur Aufspaltung der Glycolsulfatgruppen zu - CHOH - CH - $OSO_3^{(-)}$-Gruppen erwärmt.

6. Fettsäureesterderivate, wie sie erhalten werden nach dem Verfahren gemäß den Ansprüchen 1 bis 5.